# EUROPÄISCHE PATENTSCHRIFT

(11) **EP 0 692 224 B1**
(45) Veröffentlichungstag und Bekanntmachung des Hinweises auf die Patenterteilung: **29.07.1998**
(21) Anmeldenummer: 95106500.2
(22) Anmeldetag: 28.04.1995
(51) Int. Cl.: A61B 17/39, A61B 17/00, A61M 1/00

(54) **Medizinisches Multifunktionsinstrument zum endoskopischen Operieren**
Multi-function medical instrument for endoscopic operations
Instrument médical à fonctions multiples utilisé pour des opérations endoscopiques

(30) Priorität: 13.06.1994 DE 4420608
(43) Veröffentlichungstag der Anmeldung: 17.01.1996
(73) Patentinhaber: Delma elektro- und medizinische Apparatebau Gesellschaft mbH, D-78532 Tuttlingen (DE)
(72) Erfinder: Fritzsch, Gernod, D-78532 Tuttlingen (DE); Lurz, Michael, Dipl.-Ing. (FH), D-78589 Dürbheim (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(56) Entgegenhaltungen:
- EP-A- 0 327 410
- US-A- 5 254 117
- US-A- 5 265 840

## Beschreibung

Die Erfindung betrifft ein medizinisches Multifunktionsinstrument zum endoskopischen Operieren nach dem Oberbegriff des Patentanspruchs 1.

Derartige Multifunktionsinstrumente sind in vielfachen Ausführungen bekannt (siehe z.B. EP 0 327 410 A1). Ein Problem besteht darin, daß es gerade bei Multifunktionsinstrumenten darauf ankommt, daß der Operateur sie einerseits sicher halten kann, andererseits aber auch die verschiedenen Arbeitswerkzeuge sowie Saug- und Spülkanäle um die Instrumentenachse herum in eine optimale Behandlungsposition bringen kann. Die Handhabung wird dadurch noch schwieriger, daß am Bedienungshandgriff im allgemeinen verschiedene Betätigungselemente angeordnet sind, die der Operateur beaufschlagen können muß, ohne das Instrument zu verreißen.

Bei einem weiteren bekannten Multifunktionsinstrument (US-A-5 254 117) ist das mehrlumige Rohr drehbar innerhalb des Instrumentensockels angeordnet und mittels eines durch einen Schlitz des Instrumentensockels nach außen ragenden Betätigungsrades relativ zum Instrumentenkörper verdrehbar. Nachteilig an dieser Anordnung ist, daß die Bedienung durch den Operateur nicht einfach ist und daß durch den Schlitz für das Rad Schmutz in das Innere des Instrumentensockels eindringen kann. Auch ist das Instrument jedenfalls nicht einfach zerlegbar. Außerdem können keine Arbeitswerkzeuge von hinten durch den Instrumentensockel eingeschoben werden.

Das Ziel für die Erfindung besteht somit darin, ein Multifunktionsinstrument der eingangs genannten Gattung zu schaffen, bei dem trotz der exzentrischen Anordnung des Arbeitskanals bzw. mehrerer Arbeitskanäle beim endoskopischen Beobachten das bzw. die Arbeitswerkzeuge bei festgehaltenem Instrumentensockel in verschiedenen Positionen betrachtet werden können, wobei auf das problemlose Einschieben und Entnehmen eines Arbeitsinstrumentes von hinten her nicht verzichtet werden soll.

Zur Lösung dieser Aufgabe sind die Merkmale des kennzeichnenden Teils des Anspruches 1 vorgesehen.

Aufgrund dieser Ausbildung kann der Anschlußkörper bzw. das Rohr um die Drehachse herum relativ zum Instrumentensockel und insbesondere zu dem vorzugsweise pistolenartig angeordneten Bedienungshandgriff problemlos in eine solche Position verdreht werden können, daß die gegebenenfalls vorne vom Rohr vorstehenden Arbeitswerkzeuge optimal durch ein Endoskop betrachtet werden können, wobei der Exzenter eine axiale Ausrichtung des Arbeitskanals und seiner Verlängerungen in jeder Drehposition gewährleistet. Von besonderem Vorteil ist, daß nicht nur das Rohr, sondern der gesamte Instrumentenkörper relativ zum Instrumentensockel verdrehbar ist, so daß der Instrumentenkörper mit der einen Hand und der Instrumentensockel mit der anderen Hand gehalten und relativ zueinander in die gewünschte Position verdreht werden können.

Der Operateur kann daher das erfindungsgemäße Multifunktionsinstrument sicher und ergonomisch einwandfrei greifen und gleichzeitig die verschiedenen Kanäle in eine optimale Behandlungsposition bringen.

Die exzentrische Anordnung der Kanäle im Rohr ist bei der Anbringung mehrerer Arbeitskanäle sowie wenigstens eines Saug- oder Spülkanals dadurch bedingt, daß der Außendurchmesser des Rohres aus operativen Gründen auf ca. 10 mm begrenzt ist, so daß in einem relativ kleinen Querschnitt die verschiedenen Kanäle so anzuordnen sind, daß sie einen möglichst großen Durchmesser aufweisen können. Hierfür ist eine exzentrische Anordnung praktisch aller Kanäle unvermeidlich.

Eine durch Verdrehen des Instrumentenkörpers relativ zum Bedienungshandgriff einmal eingestellte Drehposition kann nach Anspruch 3 am einfachsten durch eine gewisse Reibung zwischen diesen beiden Bauteilen gesichert werden, welche gering genug ist, um eine Drehung ohne zu großen Kraftaufwand zu ermöglichen, jedoch groß genug, um ein selbstständiges Verdrehen beim normalen bestimmungsgemäßen Gebrauch des Instrumentes zu vermeiden.

Die Anordnung eines Anschlußkörpers gemäß Anspruch 4 bringt im Zusammenhang mit der Drehbarkeit den Vorteil mit sich, daß die Anschlußleitungen in Richtung zum separat aufgestellten Versorgungsgerät gedreht werden und somit den räumlichen Operationsgegebenheiten ebenfalls optimal angepaßt werden können. Weiter ist an dieser Ausführungsform vorteilhaft, daß auf die Drehung zwischen dem Instrumentenkörper und dem Bedienungshandgriff mitmachende Leitungs-Zwischenstücke verzichtet werden kann, die erforderlich wären, wenn die Anschlüsse am Instrumentensockel vorliegen würden.

Sofern am Bedienungshandgriff Betätigungselemente zum elektrischen und/oder hydraulischen und/oder pneumatischen Schalten vorgesehen sind, ist die Ausführungsform nach Anspruch 5 zweckmäßig. Auf die Schleifer-Schleifring-Kontakte kann verzichtet werden, wenn die elektrischen Steuerleitungen zur am Bedienungshandgriff angeordneten Tastatur in den Bedienungshandgriff bzw. den Instrumentensockel unmittelbar eingeführt werden. Nachdem jedoch in den Anschlußkörper bevorzugt die Fluid-Ver- bzw. -entsorgungsleitungen seitlich eingeführt werden, ist es zweckmäßig, dort auch die elektrischen Steuerleitungen ein- bzw. auszuführen, damit möglichst nur an einer Stelle des Instrumentes Leitungszu- bzw. -abführungen vorhanden sind.

Aufgrund der Weiterbildungen nach den Ansprüchen 6 bis 8 kann das Arbeitsinstrument einerseits in einer Arbeitsposition axial absolut fest mit dem Instrumentensockel bzw. dem Instrumentenkörper verbunden werden, während andererseits ein selbsttätiges Zurückschnellen in eine zurückgezogene Ruheposition durch Rückstellfederkraft bei einer Auslösung gewährleistet ist.

Die Ansprüche 9 und 10 definieren eine baulich besonders bevorzugte Ausbildung der Rast-Federanordnung zur Halterung des Arbeitsinstrumentes in der Arbeitsposition bzw. zu dessen selbsttätiger Freigabe in die Ruheposition.

Eine besonders vorteilhafte Ausführungsform des Exzenters ist durch Anspruch 11 gekennzeichnet. Anspruch 12 sieht eine weitere Funktion des Exzenters vor.

Weitere vorteilhafte Weiterbildungen der Erfindung sind im Anspruch 13 definiert.

Die Universalität des Multifunktionsinstrumentes gemäß der Erfindung wird weiter durch die Maßnahmen des Anspruches 14 erweitert.

Gemäß Anspruch 15 kann der Arbeitskanal eine Doppelfunktion als Flüssigkeits-Spül- oder Saugkanal erhalten. Das ermöglicht die Entfernung auch größerer Biogewebefragmente bzw. -konkremente oder harter Gewebeteilchen.

Das erfindungsgemäße Multifunktionsinstrument kann hinsichtlich seiner Funktionen vorteilhafterweise durch die Merkmale der Ansprüche 16 und 17 weiter verbessert werden.

Erfindungsgemäß wird also neben einem vorzugsweise großlumigen Haupt-Arbeitskanal mit in diesen zurückziehbaren Arbeitsinstrument ein weiterer, vorzugsweise kleinlumiger Arbeitskanal vorgesehen, in dem ein weiters stabförmiges Instrument in eine Ruheposition zurückziehbar und in eine Arbeitsposition ausfahrbar angeordnet ist. Hierdurch kann wahlweise das eine oder das andere Arbeitswerkzeug aus dem Rohr ausgefahren werden. Beide Arbeitskanäle sollen geradlinig ausgebildet sein.

Während das Haupt-Arbeitsinstrument vorzugsweise von Hand von hinten eingeschoben und in der Arbeitsposition verrastet wird und vorher oder anschließend gegebenenfalls in die gewünschte Position verdreht wird, ist das zweite Arbeitsinstrument normalerweise fest, jedoch herausnehmbar, im Anschlußkörper und im Rohr angeordnet und wird durch einen im Anschlußkörper vorgesehenen Antrieb, der beispielsweise pneumatisch, hydraulisch oder elektromagnetisch sein kann, durch Beaufschlagung eines Betätigungselementes der Tastatur definiert in eine Arbeitsposition ausgefahren bzw. in die im Rohr zurückgezogene Ruheposition zurückgezogen.

Die Erfindung wird im folgenden beispielsweise anhand der Zeichnung beschrieben; in dieser zeigt
- Figur 1: eine schematische Seitenansicht eines erfindungsgemäßen Multifunktionsinstrumentes, in dessen Anschlußkörper und Rohr nur der ein Arbeitsinstrument enthaltende Arbeitskanal und gestrichelt der Flüssigkeits-Spül- oder Saugkanal, nicht jedoch die übrigen Funktionsteile dargestellt sind,
- Figur 2: eine perspektivische Explosionsansicht einer bevorzugten baulichen Ausführungsform eines erfindungsgemäßen Multifunktionsinstrumentes, wobei im Inneren des Rohres und des Anschlußkörpers nur der kleinlumige zweite Arbeitskanal bzw. das darin angeordnete stabförmige Hochfrequenz-Schneid- oder -Koagulationsinstrument und sein Antrieb gestrichelt angedeutet sind,
- Figur 3: eine schematische Schnittansicht nach Linie III-III in Fig. 1,
- Figur 4: eine Rückansicht des Multifunktionsinstrumentes nach Fig. 1,
- Figur 5: eine stark vergrößerte schematische perspektivische Ansicht des distalen Endes des Rohres einer bevorzugten Ausführungsform des erfindungsgemäßen Multifunktionsinstrumentes, ohne eingesetztes Haupt-Arbeitsinstrument,
- Figur 6: eine rein schematische Seitenansicht des Fixier- und Verstellmechanismus für ein von hinten eingeschobenes Arbeitsinstrument innerhalb des Exzenters des Multifunktionsinstrumentes nach den Fig. 1 bis 5 nach dem Einschieben in der Ruheposition unmittelbar nach dem In-Eingriff-Kommen des Arbeitsinstrumentes mit den komplementären Bauelementen des Exzenters bzw. des Instrumentensockels,
- Figur 7: eine Fig. 6 entsprechende Ansicht des Fixier- und Verstellmechanismus in der weiter eingeschobenen verrasteten Arbeitsposition des Arbeitsinstrumentes, wobei die aktive Stellung des Arbeitswerkzeuges bei vorgeschobenem Führungsrohr wiedergegeben ist und
- Figur 8: eine zu Fig. 7 analoge Ansicht bei durch Ziehen am Betätigungshebel in seine inaktive Stellung überführten Arbeitsinstrument.

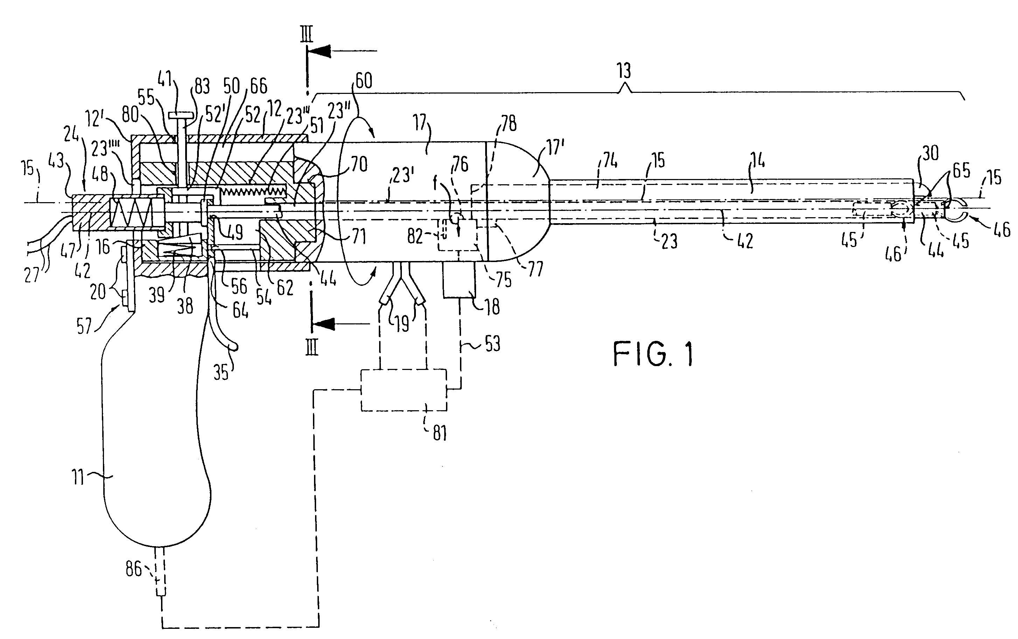

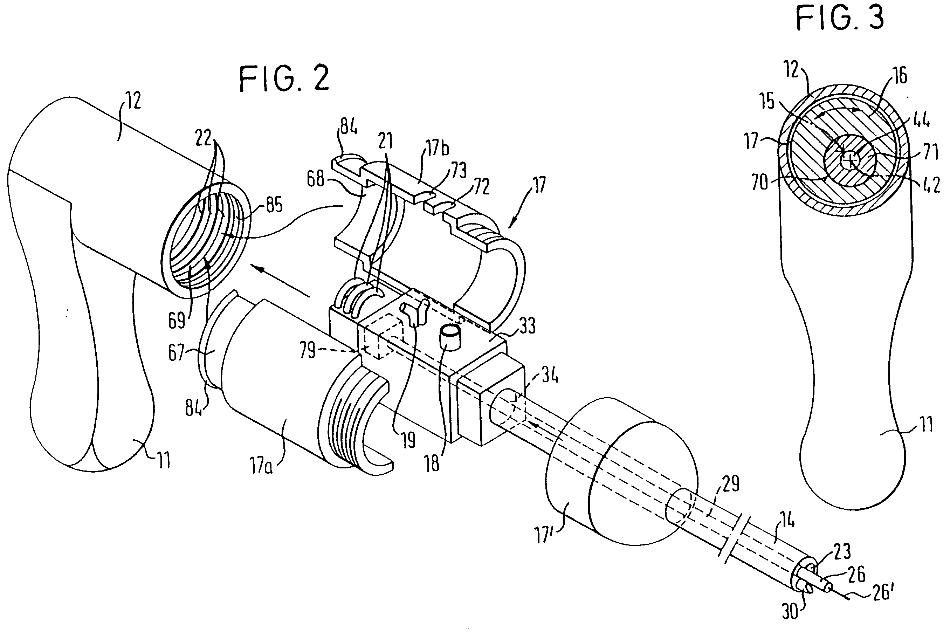

Nach Figur 1 weist ein erfindungsgemäßes Multifunktionsinstrument einen Instrumentenkörper 13 auf, der einen im wesentlichen zylindrischen Anschlußkörper 17 mit vorn darauf angeordneter Schraubkappe 17' und ein vorne aus der Schraubkappe 17' axial herausragendes mehrlumiges Kunststoff-Rohr 14 mit einer Mittelachse 15 aufweist, das nach Figur 1, 2 und 5 einen großlumigen exzentrischen ersten Arbeitskanal 23 mit einer parallel und im seitlichen Abstand zur Achse 15 verlaufenden Mittelachse 42, sowie parallel dazu einen kleinlumigen exzentrischen, zweiten Arbeitskanal 29 und einen Flüssigkeits-Spül und/oder Saugkanal 74 aufweist. In Figur 1 ist gestrichelt nur der erste Arbeitskanal 23 und der Spül- und/oder -Saugkanal 74 angedeutet. Der zweite Arbeitskanal 29 ist in Figur 1 der Übersichtlichkeit halber nicht dargestellt. In Figur 2 ist der Übersichtlichkeit halber nur der zweite Arbeitskanal 29 im Rohr 14 gestrichelt eingezeichnet, während von den beiden anderen Kanälen 23, 74 nur die Mündung bzw. eine vorn vom Rohr 14 vorstehende Verlängerung 30 gezeigt sind. Der Arbeitskanal setzt sich im Anschlußkörper 17 in eine Kanal-Verlängerung 23' fort.

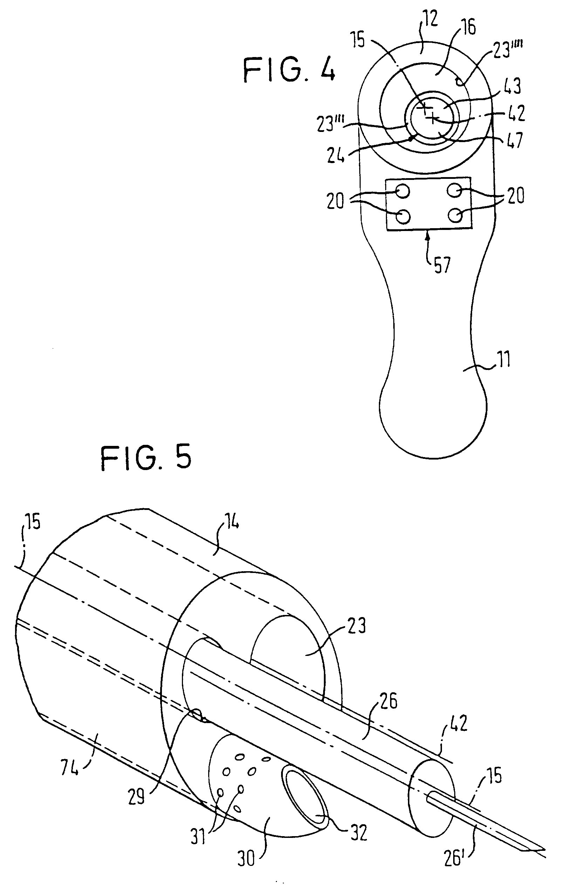

Nach Figur 1 ist in den von hinten nach vorn durchgehenden Arbeitskanal 23 des Rohres 14 ein Führungsrohr 44 eines Arbeitsinstrumentes 24 eingeführt, wobei das ein Arbeitswerkzeug 46 tragende vordere Ende gestrichelt in einer zurückgezogenen Ruheposition in ausgezogenen Linien in einer vorne aus dem Rohr 14 ausgefahrenen Arbeitsposition wiedergegeben ist. In den zweiten Arbeitskanal 29 ist nach den Figuren 2 und 5 ein stabförmiges Hochfrequenz-Schneid- und/oder Koagulationsinstrument 26 eingebaut, dessen mit einer vorne vorstehenden Schneidnadel 26' versehenes distales Ende aus der Vorderfläche des Rohres 14 vorsteht und so eine Arbeitsposition einnimmt, aus der es jedoch in der weiter unten im einzelnen beschriebenen Weise nach hinten in den zweiten Arbeitskanal 29 zurückgezogen werden kann, so daß der vordere Teil des Instrumentes 26 einschließlich der Schneidnadel 26' vollständig im zweiten Arbeitskanal 29 verschwindet.

Der Anschlußkörper 17 ist um die Achse 15 drehbar an einem ebenfalls im wesentlichen zylindrisch ausgebildeten Instrumentensockel 12 befestigt, von dem sich hinten im wesentlichen im rechten Winkel zur Mittelachse 15 ein Bedienungshandgriff 11 wegerstreckt, der an seiner Rückseite (Fig. 1, 4) zu einer Tastatur 57 zusammengefaßte Betätigungselemente 20 für elektrische und/oder hydraulische und/oder pneumatische Steuerungen aufweist. Im Anschlußkörper 17 münden nach Fig. 1, 2 radial eine Fluid-Versorgungs- bzw. Entsorgungsleitungsanordnung 18 und elektrische Steuer- und/oder Versorgungskabel 19, die zu einem in Figur 1 nur gestrichelt angedeuteten Steuergerät 81 führen. Auch die Fluid-Ver- bzw. Entsorgungsleitungsanordnung 18 ist in der in Figur 1 gestrichelt angedeuteten Weise über eine Verbindungsleitung 53 mit dem zentralen Steuergerät 81 verbunden.
Die Fluid-Ver- bzw. Entsorgungsleitungsanordnung 18 ist im Inneren des Anschlußkörpers 17 mit einem in Figur 1 gestrichelt dargestellten Umschaltventil 75 verbunden, welches wahlweise eine Verbindung zwischen der Fluid-Versorgungs- bzw. -Entsorgungsleitungsanordnung 18 einerseits und über eine Anschlußleitung 78 mit dem im Anschlußkörper 17 und dem Rohr 14 vorgesehenen Spül- und/oder Saugkanal 74 oder über eine Anschlußleitung 77 mit dem Inneren des ersten Arbeitskanals 23 bzw. seiner Verlängerung 23' andererseits herzustellen gestattet. Wenn kein Arbeitsinstrument 24 im ersten Arbeitskanal 23 bzw. seiner Verlängerung 23' untergebracht ist, steht in der in Figur 1 gestrichelt angedeuteten Weise ein Betätigungsvorsprung 76 des Umschaltventils 75 in die Verlängerung 23' des Arbeitskanals 23 vor, welcher durch das eingeschobene Arbeitsinstrument 24 in Richtung des Pfeiles f zurückgeschoben wird, wodurch die zunächst vorhandene Flüssigkeitszufuhr bzw. -abfuhr zwischen der Leitungsanordnung 18 und der Anschlußleitung 78 unterbrochen und zwischen der Leitungsanordnung 18 und der Anschlußleitung 77 geöffnet wird. Mit anderen Worten wird in allen Fällen, wo sich kein Arbeitsinstrument 24 im Arbeitskanal 23 befindet letzterer als Spül- und/oder Saugkanal verwendet. Damit im letzteren Fall der Arbeitskanal 23 nach hinten verschlossen ist, gelangt nach dem Herausziehen des Arbeitsinstrumentes 24 aus dem Arbeitskanal 23 bzw. seiner Verlängerung 23' im Anschlußkörper 17 ein Schließglied 82 das Teil des Ventilmechanismus sein kann, automatisch in die Kanal-Verlängerung 23', so daß über die Anschlußleitung 77 eintretende Flüssigkeit gezwungen ist, zum distalen Ende des Arbeitskanals 23 zu strömen.

Der Spül- und/oder Saugkanal 74 mündet am distalen Ende des Kunststoff-Rohres 14 nach Figur 5 in die kurze rohrartige Verlängerung 30, die seitlich Entlastungsbohrungen 31 aufweist und deren Mündung 32 unter einem solchen Winkel zur Mittelachse 15 angeordnet ist, daß ein dort austretender Spülstrahl auf diejenigen Bereiche gerichtet ist, die von einem der in ihrer Arbeitsposition befindlichen Arbeitswerkzeuge 26', 46 behandelt werden sollen.

Nach den Figuren 1, 3 und 4 ist im Instrumentensockel 12 konzentrisch zur Mittelachse 42 des ersten Arbeitskanals 23, d. h. exzentrisch zur Drehachse 15 ein im wesentlichen zylindrischer hohl ausgebildeter Exzenter 16 angeordnet, welcher vorne einen in eine hintere exzentrische Aufnahmebohrung 70 des Anschlußkörpers 17 drehbar eingreifenden, konzentrischen Führungszapfen 71 aufweist und mit seiner hinteren Stirnfläche an der Rückwand 12' des Instrumentensockels 12 gleitend anliegt. U. a. durch den durch eine Radialbohrung 80 hindurchtretenden radialen Stößel 83 eines weiter unten zu beschreibenden Auslöseknopfes 41, welcher durch eine Radialbohrung 55 in den Instrumentensockel 12 eintritt, wird der Exzenter 16 gegen eine wesentliche Verdrehung innerhalb des Instrumentensockels 12 gehalten. Er kann sich jedoch innerhalb eines im Hohlraum des Instrumentensockels 12 vorgesehenen Spielraums 66 derart radial verschieben, daß die mit der Längsmittelachse 42 des Arbeitskanals 23 identische Mittelachse des Exzenters 16 um die Drehachse 15 herum eine Umlaufbewegung ausführen kann, ohne daß der Exzenter 16 sich selbst wesentlich dreht.

Der Arbeitskanal 23 des Rohres 14 und seine Verlängerung 23' gehen hinten axial in eine entsprechend dimensionierte Verlängerung 23'' innerhalb des Führungszapfens 71 über, an die sich eine weitere Verlängerung des Arbeitskanals 23 darstellender Hohlraum 23''' innerhalb des Exzenters 16 axial anschließt. Hinten endet die Verlängerung 23''' in eine großdimensionierte Öffnung 23'''' in der Rückwand 12' des Instrumentensockels 12 über.

Bei einer Drehung des das Rohr 14 tragenden Anschlußkörpers 17 um die Mittelachse 15 des Rohres 14 und des Anschlußkörpers 17 rotiert die Mittelachse 42 der Aufnahmebohrung 70 um die Drehachse 15 herum und nimmt dabei den Exzenter 16 zu einer entsprechenden Umlauf-Taumelbewegung mit. Dadurch bleiben der Arbeitskanal 23 und seine Verlängerung 23' im Anschlußkörper 17 mit den weiteren Verlängerungen 23'' und 23''' im Exzenter 16 ständig ausgerichtet, so daß in jeder Drehposition des Anschlußkörpers 17 von hinten durch die Öffnung 23'''' ein stabförmiges Arbeitsinstrument 24 in die aus Figur 1 ersichtliche Position innerhalb der Arbeitskanalanordnung 23, 23', 23'', 23''', 23'''' eingeschoben werden kann.

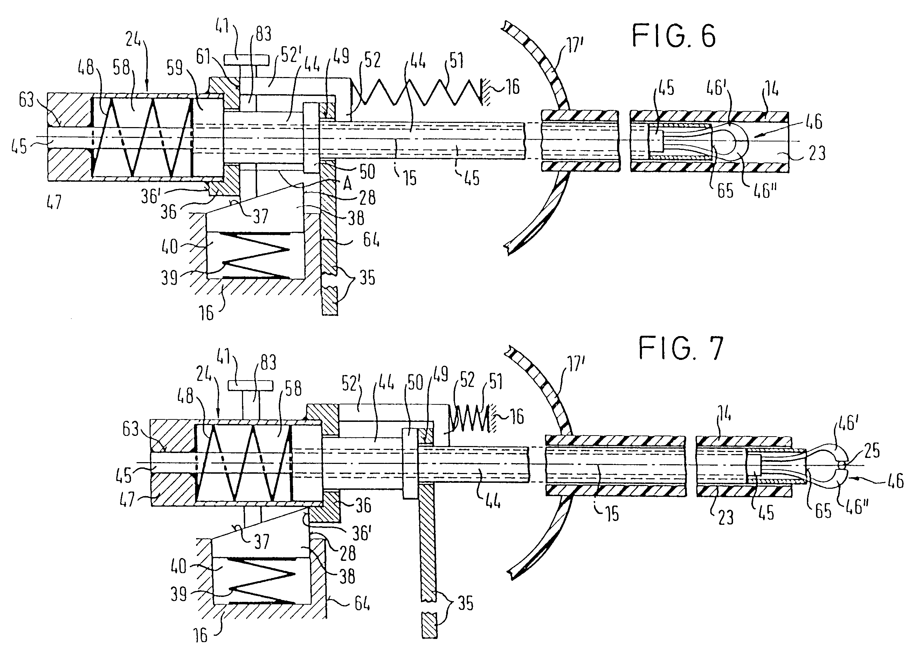

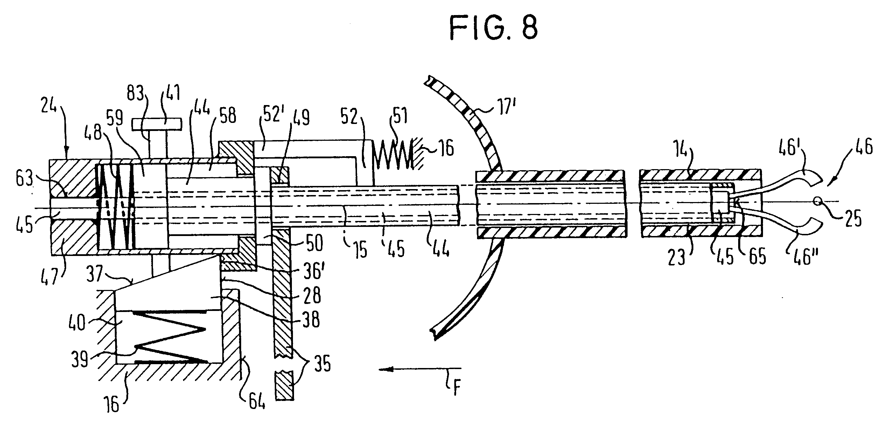

Das proximale Ende 43 des Arbeitsinstrumentes 24 steht durch die zentrale Öffnung 23'''' vom hinteren Ende des Instrumentensockels 12 vor und dient zum Anbringen etwaiger elektrischer und/oder hydraulischer und/oder pneumatischer Versorgungs- bzw. Entsorgungsleitungen 27. Im vorliegenden Ausführungsbeispiel ist an die Anschlußleitungen 27 eine Koagulations-Hochfrequenzspannung anlegbar, welche durch in einem zentralen Arbeitsstab 45 des Arbeitsinstrumentes 24 verlegte Leitungen in weiter unten anhand von Figur 6 und 8 erläuterter Weise zu zwei das Arbeitswerkzeug 46 bildenden Koagulations-Klemmelektroden 46', 46'' am vorderen Arbeitsende geleitet ist.

Nach Fig. 2 ist der Anschlußkörper 17 aus zwei Halbschalen 17a, 17b zusammengesetzt, welche auf der einen Seite durch Einsetzen in den rohrförmig ausgebildeten Instrumentensockel 12 und auf der anderen Seite durch die aufgesetzte Schraubkappe 17' zusammengehalten werden. Zwischen dem Instrumentensockel 12 und den dort axial eingesetzten Halbschalen 17a, 17b liegt eine Drehverbindung vor, wozu sich vom hinteren Ende der Halbschalen 17a, 17b halbzylinderförmige Zapfenteile 67, 68 in eine zentrale konzentrische Bohrung 69 am vorderen Ende des Instrumentensockels 12 eingreifen. Die axiale Fixierung der in den Instrumentensockel 12 eingesetzten Gehäusehalbschalen 17a, 17b wird durch am Umfang der Zapfenteile 67, 68 vorgesehene Wülste 84 erreicht, die in dazu komplementäre Ausnehmungen 85 am Innenumfang des hohlzylindrisch ausgebildeten Instrumentensockels 12 im Bereich der Aufnahmeöffnung 69 eingreifen. Die Halbschalen 17a, 17b schließen zwischen sich ein Anschlußteil 33 fest ein, welches eine Axialbohrung 34 für die Aufnahme des vorzugsweise abnehmbar ausgebildeten Rohres 14 aufweist und außerdem federnde Schleifkontakte 21 trägt, die nach dem Zusammenbau des Instrumentes mit an der Innenwand des rohrförmig ausgebildeten Instrumentensockels 12 vorgesehenen Schleifringen 22 in elektrisch leitenden Kontakt kommen. Auf diese Weise können auch bei Verdrehung des aus den Halbschalen 17a, 17b gebildeten Anschlußkörper 17 die elektrischen Steuer- und Schaltfunktionen von den Betätigungselementen 20 am Bedienungshandgriff 11 auf das Anschlußteil 33 übertragen werden.

In der Gehäusehalbschale 17b befinden sich zu einem Rand hin offene Durchlaßschlitze 72, 73, welche komplementär zu den vom Anschlußteil 33 vorstehenden Fluid-Versorgungs- bzw. Entsorgungsleitungen 18 bzw. den elektrischen Versorgungs- und Steuerleitungen 19 ausgebildet sind, so daß nach dem Zusammenbau die Leitungen 18, 19 aus den Durchlaßschlitze 72, 73 radial vorstehen.

Sofern die Betätigungselemente 20 (Fig. 1, 4) auch hydraulische und/oder pneumatische Schalter oder Regelungselemente umfassen sollten, können sich vom Instrumentensockel 12 in den drehbaren Anschlußkörper 17 auch hydraulische Leitungen erstrecken, die so verlegt bzw. biegsam angeordnet sind, daß sie auch bei einer Relativverdrehung zwischen Instrumentensockel 12 und Anschlußkörper 17 ihre Funktion ausüben können. Grundsätzlich ist es auch denkbar, daß die mit den Betätigungselementen 20 der Tastatur 57 in Verbindung stehenden Steuerleitungen bei 86 in Figur 1 aus dem Bedienungshandgriff 11 herausgeführt und von dort zum Steuergerät 81 geführt sind.

Eine besonders bevorzugte Ausführungsform eines Arbeitsinstrumentes 24 weist nach den Figuren 1 und 6 bis 8 ganz hinten ein kreiszylindrisches Basisteil 47 auf, welches an seinem vorderen Ende einen zylindrischen Hohlraum 58 aufweist, in dem ein von einer Feder 48 nach vorn beaufschlagter Kolben 59 angeordnet ist, von dem sich nach vorne das Führungsrohr 44 erstreckt, welches durch den Arbeitskanal 23 und seine Verlängerungen 23', 23'', 23''' hindurchpaßt sowie dort drehbar und axial verschiebbar gehalten ist. Durch das Führungsrohr 44, den Kolben 59, die Feder 48 und gegebenenfalls eine entsprechende Axialbohrung 63 im Basisteil 47 erstreckt sich ein mit dem Basisteil 47 fest verbundener, relativ zum Führungsrohr 44 axial beweglicher Arbeitsstab 45 bis zum vorderen Rand 65 des Führungsrohres 44, wo von dem Arbeitsstab 45 das Arbeitswerkzeug 46 nach vorne vorsteht, welches aus den beiden klemmbaren Elektroden 46', 46'' eines Hochfrequenz-Koagulationsinstrumentes besteht.

Am vorderen Ende des Basisteils 47 ist ein Rastkragen 36 vorgesehen, während das Führungsrohr 44 in einem Abstand A (Fig. 6) vor dem Rastkragen 36 (bei in der vordersten Stellung befindlichem Kolben 59) einen Anschlagkragen 50 trägt.

Der Anschlagkragen 50 arbeitet nach den Fig. 6 bis 8 mit einem querverschieblich im Exzenter 16 angeordneten Rastelement 38 zusammen, welches durch eine Ausstellfeder 39 radial nach innen auf den Rastkragen 36 zu vorgespannt ist und an seiner radial inneren Seite eine schiefe Ebene 37 trägt, an der der Rastkragen 36 beim Einschieben des Arbeitsinstrumentes 24 von hinten in die Arbeitskanalanordnung 23, 23', 23'', 23''', 23'''' gemäß den Fig. 6 und 7 hochläuft und dabei das Rastelement 38 innerhalb einer im Exzenter 16 vorgesehenen Radialführung 40 radial nach außen gegen die Kraft der Feder 39 verschiebt.

Sobald der Rastkragen 36 das vordere Ende des Rastelements 38 erreicht hat, schnappt dieses unter der Wirkung der Feder 39 gemäß Fig. 7 hinter die hintere senkrecht zur Mittelachse 15 verlaufende Stirnfläche 36' des Rastkragens 36, so daß die vordere und ebenfalls senkrecht zur Mittelachse 15 verlaufende Endfläche 28 des Rastelementes 38 mit der Stirnfläche 36' in einem Eingriff steht, der ein Bewegen des Arbeitsinstrumentes 24 nach hinten unmöglich macht, bis das Rastelement 38 durch radiales Drücken eines von außen zugänglichen Auslöseknopfes 41 (Figur 1, 6 bis 8), der über den radialen Stößel 83 mit dem Rastelement 38 verbunden ist, wieder außer Eingriff mit dem Rastkragen 36 gebracht wird. Der Stößel 83 erstreckt sich durch die Radialbohrung 55 in der Umfangswand des Instrumentensockels 12 mit soviel Winkelspiel, daß er den bei der Umlauf-Taumelbewegung des Exzenters 16 auftretenden Kippbewegungen folgen kann.

Die Begrenzung der Bewegung des Arbeitsinstrumentes 24 nach vorn wird dadurch herbeigeführt, daß der Rastkragen 36 mit seiner Frontfläche 61 gegen einen axialen Fortsatz 52' eines Anschlagkragen-Eingriffhalters 52 anstößt, der durch eine Feder 51 nach vorn am Exzenter 16 abgestützt ist und Bestandteil des Exzenters 16 ist.

Auf diese Weise schiebt der Rastkragen 36 beim Hochlaufen auf der schiefen Ebene 37 und dem radialen Nach-Außen-Schieben des Rastelementes 38 gleichzeitig den Anschlagkragen-Eingriffshalter 52 unter Zusammendrückung der Feder 51 nach vorn in die aus Fig. 7 ersichtliche vordere End-Position. Auf diese Weise wird das Arbeitsinstrument 24 in der aus Fig. 7 ersichtlichen Arbeitsstellung nach vorn durch die entsprechend zusammengedrückte Feder 51 und nach hinten durch das Rastelement 38 fixiert.

Nach den Fig. 1, 6 und 7 ist vor dem Anschlagkragen 50 auf dem Führungsrohr 44 mittels einer entsprechend dimensionierten Bohrung 49 ein Betätigungshebel 35 angeordnet, der nach Fig. 1 aus einer entsprechenden Aussparung 62 des Exzenters 16 und des Instrumentensockels 12 parallel zum Bedienungshandgriff 11 nach unten herausragt, und zwar in der Weise, daß er beim Ergreifen des Bedienungshandgriffes 11 von dem Zeigefinger der Bedienungsperson nach Art des Abzugs einer Pistole ergriffen werden kann.

Während nach Fig. 6 der Betätigungshebel 35 an seinem im Exzenter 16 befindlichen Endbereich von hinten vom Anschlagkragen 50 beaufschlagt wird, greift von vorn der von der Feder 51 nach hinten beaufschlagte Anschlag-Eingriffshalter 52 an ihm an, wodurch der Betätigungshebel 35 in der aus Fig. 6 ersichtlichen Position zwischen den Bauelementen 50, 51 federnd gehalten ist. Die Feder 51 spannt den Betätigungshebel 35 über den Anschlagkragen-Eingriffshalter 52 gegen eine Anschlagfläche 64 des Exzenters 16 vor, so daß der Betätigungshebel 35 bei herausgenommenen Arbeitsinstrument 24 und in der Ruheposition nach Figur 1 und 6 an der Anschlagfläche 64 anliegt.

Der Betätigungshebel 35 ist außerdem in in Fig. 1 nur angedeuteter Weise am Instrumentensockel 12 axial verschieblich geführt. In Fig. 1 ist zu diesem Zweck in der Aussparung 62 eine sockelfeste Führungsstange 54 angedeutet, die durch eine dazu komplementäre Führungsbohrung 56 im Betätigungshebel 35 hindurchgreift.

Nach Figur 2 erstreckt sich der zweite kleinere Arbeitskanal 29 innerhalb des Rohres 14 und des Anschlußteils 33 des Anschlußkörpers 17 bis zu einem nur gestrichelt schematisch angedeuteten Antrieb 79, welcher beispielsweise eine pneumatische Kolben-Zylinderanordnung sein kann, mit der das in den Arbeitskanal 29 von vorn eingesteckte stabförmige Arbeitsinstrument 26 mit der Hochfrequenz-Schneidnadel 26' derart in Eingriff gebracht werden kann, daß bei entsprechender Betätigung des Antriebes 79 durch Beaufschlagung der dafür vorgesehenen Druckknöpfe 20 der Tastatur 57 (Figuren 1, 4) das Arbeitsinstrument 26 wahlweise aus dem vorderen Ende des Rohres 14 ausgefahren bzw. in dieses zurückgezogen werden kann.

Die Funktion des Arbeitsinstrumentes 24 wird im folgenden anhand der Fig. 1 und 6 bis 8 beschrieben:

Bei nicht eingesetztem Arbeitsinstrument 24 drückt die Feder 51 den Betätigungshebel 35 über den Anschlagkragen-Eingriffshalter 52 in die aus den Fig. 1 und 6 ersichtliche hinterste Position, wo der Betätigungshebel 35 beispielsweise an einer Anschlagfläche 64 des Exzenters 16 von vorn anliegt.

Wird nunmehr von hinten das Arbeitsinstrument 24 in die Arbeitskanalanordnung 23, 23', 23'', 23''', 23'''' eingeschoben, so gelangt das Führungsrohr 44 durch die Bohrung 49 des Betätigungshebels 35 hindurch in die aus den Fig. 1 und 6 ersichtliche Position, wo der Rastkragen 36 in Anlage an dem Fortsatz 52' gekommen und auch der Anschlagkragen 50 von hinten in Eingriff mit dem Betätigungshebel 35 gekommen ist. Dies ist die auch in Figur 1 dargestellte Ruheposition des Arbeitsinstrumentes 24, in der das Arbeitswerkzeug 46 gemäß Figur 6 und der gestrichelten Darstellung in Figur 1 in das vordere Ende des Rohres eingezogen ist.

Wird nun das Arbeitsinstrument 24 weiter im Instrumentensockel 12 bzw. Exzenter 16 nach vorne geschoben, so nimmt der Anschlagkragen 50 über den Betätigungshebel 35 den Anschlagkragen-Eingriffshalter 52 unter Zusammendrückung der Rückstellfeder 51 mit, wobei der Rastkragen 36 an der schiefen Ebene 37 des Rastelements 38 emporgleitet und dabei das Rastelement 38 innerhalb seiner Radialführung 40 zurückschiebt, bis es schließlich in die aus Figur 7 ersichtliche Arbeitsposition schnappt. Während des Vorschiebens des Arbeitsinstrumentes 24 aus der Ruheposition von Figur 6 in die Arbeitsposition von Figur 7 tritt das zunächst in das vordere Ende des Rohres 14 zurückgezogene Arbeitswerkzeug 46 aus der in Figur 6 wiedergegebenen und in Figur 1 gestrichelt dargestellten Position in die aus Figur 7 ersichtliche und in Figur 1 in ausgezogenen Linien dargestellte Arbeitsposition aus dem vorderen Ende des Rohres 14 aus. In dieser Position ist der Betätigungshebel 35 nach wie vor zwischen dem Anschlagkragen 50 und dem Anschlagkragen-Eingriffshalter 52 eingespannt und zwar aufgrund der Wirkung der nunmehr maximal zusammengedrückten Rückstellfeder 51.

Bei der Verschiebung von der Ruheposition nach Figur 6 in die Arbeitsstellung nach Fig. 7 ist also der Betätigungshebel 35 nach vorne mitgenommen worden, so daß er nunmehr gemäß Fig. 8 durch Zurückziehen mit dem Finger in Richtung des Pfeiles F unter Mitnahme des Führungsrohres 44 nach hinten verschoben werden kann. Dabei wird das Führungsrohr 44 mit dem Kolben 59 unter Zusammendrückung der Feder 48 nach hinten mitgenommen. In dieser Position nehmen die federnden Klemmelektroden 46', 46'' die aus Fig. 8 ersichtliche entklemmte Position ein, so daß die Elektroden 46', 46'' beispielsweise über ein nur schematisch angedeutetes Gewebeteil 25 geführt werden können.

Wird nunmehr die in Richtung des Pfeiles F auf den Betätigungshebel 35 ausgeübte Fingerkraft in geeignetem Maße reduziert, so verschiebt die beim Zurückziehen des Bedienungshebels 35 zusammengedrückte Feder 48 den Kolben 59 und damit das Führungsrohr 44 wieder nach vorne, wobei der vordere Rand 65 des Führungsrohres 44 an den leicht federnd nach außen gespreizten Elektroden 46', 46'' entlanggleitet und diese dadurch gemäß der Darstellung nach Fig. 7 zangenartig zusammenspannt. Dadurch wird das Gewebeteil 25 geklemmt und kann nunmehr durch Einschalten eines Hochfrequenz-Koagulationsstromes in der gewünschten Weise koaguliert werden. Die Klemmung kann durch erneutes Zurückziehen des Betätigungshebels 35 in die Position nach Fig. 8 aufgehoben werden.

Soll das Arbeitsinstrument 24 aus der unbetätigten Arbeitsposition nach Fig. 7 wieder in die zurückgezogene Ruheposition nach Fig. 6 überführt werden, so wird der Auslöseknopf 41 gedrückt, wodurch das Rastelement 38 mit dem Rastkragen 36 außer Eingriff kommt und die Feder 51 über den Anschlagkragen-Eingriffshalter 52 und dessen Fortsatz 52' das Arbeitsinstrument 24, dessen Arbeitswerkzeug 46 bis dahin gemäß Fig. 7 aus dem vorderen Ende des Rohres 14 vorgestanden hatte, wie das auch in Figur 1 in ausgezogenen Linien dargestellt ist, in die in Figur 1 gestrichelt angedeutete Rückzugsposition innerhalb des Arbeitskanals 23 gemäß Fig. 6 zurückgezogen wird. In dieser Ruheposition kann das Arbeitsinstrument 24 verbleiben, bis es für eine weitere Behandlung benötigt wird. Es kann aus dieser entspannten Rückzugslage aber auch nach hinten aus dem Instrumentensockel 12 und dem Instrumentenkörper 13 vollständig herausgezogen werden, wobei das Betätigungselement 76 (Fig. 1) freigegeben und dadurch automatisch der nunmehr freie Arbeitskanal 23 durch Schließen mittels des Schiebers 82 und Anschluß über die Leitung 77 an die Fluid-Versorgungs- bzw. Entsorgungsleitung 18 als Saug- oder Spülkanal verwendet werden kann.

Das in den Instrumentensockel 12 und den Instrumentenkörper 13 gemäß Figur 1 eingesetzte Arbeitsinstrument 24 kann ebenso wie der Anschlußkörper 17 selbst im Sinne des Doppelpfeiles 60 in Figur 1 in beiden Richtungen in eine gewünschte Stellung verdreht werden.

Aufgrund der erfindungsgemäßen Ausbildung können folgende Arbeiten mit dem erfindungsgemäßen Multifunktionsinstrument ausgeführt werden:

Bei nicht eingesetztem Arbeitsinstrument 24 wird der große Arbeitskanal 23 als Saug- oder Spülkanal verwendet, während das Arbeitsinstrument 26 mit der Schneidnadel 26' durch Betätigung des Antriebes 79 mittels der zugeordneten Tasten 20 der Tastatur 57 (Fig. 2) in die Arbeitsposition nach Figur 5 ausgefahren werden kann. Der normale Saug- oder Spülkanal 74 ist in diesem Fall außer Funktion gesetzt.

Das Arbeitsinstrument 24 kann während des vorstehend beschriebenen Arbeitsvorganges aber bereits in das Instrument in die aus Figur 1 gezeigte Ruheposition eingeschoben sein, in der sich das Arbeitswerkzeug 46 in der gestrichelt dargestellten zurückgezogenen Position innerhalb des Arbeitskanals 23 befindet. In diesem Fall ist die Fluid-Ver- bzw. Entsorgungsleitung 18 vom Arbeitskanal 23 durch das Ventil 75 abgeschaltet und an den kleineren Saug- oder Spülkanal 74 angeschlossen, welcher auf diese Weise seine Funktion über die Rohrverlängerung 30 nach Figur 5 ausüben kann.

Soll nun das Arbeitsinstrument 46 wirksam werden, so wird zunächst durch Betätigung des Antriebes 79 (Fig. 2) mittels der entsprechenden Taste 20 das Arbeitsinstrument 26 in den Arbeitskanal 29 zurückgezogen und dann durch Einschieben und Einrasten des Arbeitsinstrumentes 24 in die aus Figur 7 ersichtliche Position das Arbeitswerkzeug 46 vorn aus dem Rohr 14 ausgefahren. Durch Verdrehen des Anschlußkörpers 17 kann dann das Arbeitsinstrument 46 in die gewünschte Position relativ zur Drehachse 15 gebracht werden. Weiter kann das Arbeitsinstrument 24 auch um seine Achse 42 in die gewünschte Drehstellung gebracht werden.

Durch Ziehen an dem Betätigungshebel 35 in die aus Figur 8 ersichtliche Position wird das Führungsrohr 44 zurückgezogen, wodurch dessen vorderer Rand 65 an den Außenkanten der beiden Branchen 46', 46'' zurückgleitet und diese aufgrund der ihnen innewohnenden Federkraft auseinandergespreizt werden, um dadurch über ein Gewebeteil 25 greifen zu können.

Wird dann der Betätigungshebel 35 losgelassen, gelangt er aufgrund der Wirkung der Feder 48 in die aus Figur 7 ersichtliche Position, in welcher das Gewebeteil 25 durch Zusammendrücken der Branchen 46', 46'' mittels des Vorderrandes 65 des durch die Wirkung der Feder 48 vorgeschobenen Führungsrohres 44 ergriffen wird.

### Bezugszeichenliste

- 11: Bedienungshandgriff
- 12: Instrumentensockel
- 12': Rückwand
- 13: Instrumentenkörper
- 14: Rohr
- 15: Längsmittelachse (Drehachse)
- 16: Exzenter
- 17: Anschlußkörper
- 17': Schraubkappe
- 17a: Halbschale
- 17b: Halbschale
- 18: Fluid-Versorgungs- bzw. Entsorgungsleitung
- 19: elektrische Versorgungs- und Steuerleitungen
- 20: Betätigungselemente
- 21: Schleifkontakte
- 22: Schleifringe
- 23: Arbeitskanal
- 23': Verlängerung des Arbeitskanals im Anschlußkörper 17
- 23'': vordere Verlängerung d. Arbeitskanals im Exzenter 16
- 23''': hintere Verlängerung d. Arbeitskanals im Exzenter 16
- 23'''': Öffnung in der hinteren Stirnwand des Instrumentensockels 12
- 24: Arbeitsinstrument
- 25: Gewebeteil
- 26: Hochfrequenz-Schneid- oder Koagulationsinstrument
- 26': Schneidnadel
- 27: Anschlußleitungen
- 28: Endfläche
- 29: zweiter Arbeitskanal
- 30: Verlängerung
- 31: Entlastungsbohrungen
- 32: Mündung
- 33: Anschlußteil
- 34: Axialbohrung
- 35: Betätigungshebel
- 36: Rastkragen
- 36': Stirnfläche
- 37: schiefe Ebene
- 38: Rastelement
- 39: Ausstellfeder
- 40: Radialführung
- 41: Auslöseknopf
- 42: Längs-Mittelachse des Arbeitskanals 23
- 43: proximales Anschluß- bzw. Betätigungsende
- 44: Führungsrohr
- 45: Arbeitsstab
- 46: Arbeitswerkzeug
- 46': Klemmelektrode
- 46'': Klemmelektrode
- 47: Basisteil
- 48: Feder
- 49: Bohrung
- 50: Anschlagkragen
- 51: Rückstellfeder
- 52: Anschlagkragen-Eingriffshalter
- 52': Fortsatz
- 53: Verbindungsleitung
- 54: Führungsstange
- 55: Bohrung
- 56: Führungsbohrung
- 57: Tastatur
- 58: Hohlraum
- 59: Kolben
- 60: Doppelpfeil
- 61: Frontfläche
- 62: Aussparung
- 63: Bohrung für Arbeitsstab 45 im Basisteil 47
- 64: Anschlagfläche
- 65: vorderer Rand
- 66: Spielraum
- 67: Zapfenteil
- 68: Zapfenteil
- 69: Öffnung
- 70: Aufnahmebohrung
- 71: Führungszapfen
- 72: Durchlaßschlitz
- 73: Durchlaßschlitz
- 74: Flüssigkeits-Spühl- oder -Saugkanal
- 75: Umschaltventil
- 76: Betätigungselement
- 77: Anschlußleitung
- 78: Anschlußleitung
- 79: Antrieb
- 80: Radialbohrung
- 81: Steuergerät
- 82: Schließglied
- 83: Stößel
- 84: Wülste
- 85: Ausnehmung
- 86: Anschlußleitungen

## Patentansprüche

1. Multifunktionsinstrument zum endoskopischen Operieren mit einem einen Bedienungshandgriff (11) tragenden Instrumentensockel (12), an dem ein Instrumentenkörper (13) mit einem durch ein Trokar in eine Körperhöhle eines Patienten einführbaren mehrlumigen Rohr (14) angebracht ist, das vorzugsweise aus elektrisch nichtleitendem Material besteht und wenigstens einen axialen Arbeitskanal (23) aufweist, der durch komplementäre Kanäle bildende axiale Verlängerungen (23', 23'', 23''') im Instrumentensockel (12) axial verlängert ist, in den ein stabförmiges Arbeitsinstrument (24) mit einem am distalen Ende vorgesehenen Arbeitswerkzeug (46) und einem hinten vorzugsweise von dem Instrumentensockel (12) vorstehenden proximalen Anschluß- bzw. Betätigungsende (43) vorzugsweise von hinten einschiebbar und in der Arbeitsposition festlegbar ist, und dessen Längsmittelachse (42) zur Mittelachse (15) des Rohres (14) exzentrisch angeordnet ist, dadurch
**gekennzeichnet,**
daß der Instrumentenkörper (13) um eine seiner Längsachsen, vorzugsweise die Mittelachse (15) des Rohres (14) relativ zu dem mit dem Bedienungshandgriff (11) fest verbundenen Instrumentensockel (12) drehbar und in der eingestellten Drehposition haltbar ist und daß eine axiale Verlängerung (23'', 23''') des Arbeitskanals (23) in einem innerhalb des Instrumentensockels (12) mit radialem Spiel (66), axial jedoch fest angeordneten, mit der Achse (42) der Verlängerungen (23'', 23''') um die Drehachse (15) des Instrumentenkörpers (13) herumführbaren, um die Achse (42) des Arbeitskanals (23) jedoch im wesentlichen drehfest gehaltenen Exzenter (16) derart exzentrisch angeordnet ist, daß die Verlängerungen (23'', 23''') im Exzenter (16) bei jeder Drehposition des Instrumentenkörpers (13) relativ zum Instrumentensockel (12) in axialer Ausrichtung mit dem Arbeitskanal (23) bzw. seiner Verlängerung (23') im Anschlußkörper (17) bringbar sind, wobei die im Exzenter (16) vorgesehenen Verlängerungen (23'', 23''') in jeder Drehstellung des Exzenters (16) durch eine Öffnung (23'''') in der hinteren Stirnseite des Instrumentensockels (12) zugänglich sind und wobei die Achse (42) des Arbeitskanals (23) einerseits und die Achse der Verlängerungen (23'', 23''') im Exzenter (16) andererseits bei jeder Drehposition des Instrumentenkörpers (13) in axialer Ausrichtung gehalten sind.

2. Multifunktionsinstrument nach Anspruch 1,
dadurch **gekennzeichnet,**
daß der Bedienungshandgriff (11) sich zumindest im wesentlichen quer zur Längsmittelachse (15) des Instrumentenkörpers (13) bzw. des Rohres (14) erstreckt.

3. Multifunktionsinstrument nach Anspruch 1 oder 2,
dadurch **gekennzeichnet,**
daß die Drehverbindung zwischen dem Instrumentensockel (12) und dem Instrumentenkörper (13) schwergängig ist, derart, daß eine Drehverstellung möglich, die eingestellte Drehposition jedoch bei bestimmungsgemäßem Gebrauch beibehalten wird.

4. Multifunktionsinstrument nach einem der vorhergehenden Ansprüche,
dadurch **gekennzeichnet,**
daß der Instrumentenkörper (13) einen an dem Instrumentensockel (12) drehbar angebrachten Anschlußkörper (17), von dem vorzugsweise seitlich Versorgungs- bzw. Entsorgungsleitungen und/oder Steuerleitungen (18, 19) abzweigen, und an seinem vom Instrumentensockel (12) abgewandten Ende das vorzugsweise lösbar angebrachte Rohr (14) aufweist und daß eine axiale Verlängerung (23') des Arbeitskanals (23) im Anschlußkörper (17) des Instrumentenkörpers (13) zur axialen Drehachse (15) des Anschlußkörpers (17) bzw. des Rohres (14) exzentrisch angeordnet ist, wobei der Anschlußkörper (17) und der Exzenter (16) vorzugsweise so drehgekoppelt sind, daß die in ihnen vorgesehenen Verlängerungen (23', 23'', 23''') des Arbeitskanals (23) stets axial ausgerichtet sind.

5. Multifunktionsinstrument nach einem der vorangehenden Ansprüche,
dadurch **gekennzeichnet,**
daß sich am Bedienungsgriff (11) bzw. Instrumentensockel (12) ohne Lockerung des festen Griffes, beispielsweise mit dem Daumen oder Zeigefinger bedienbare Betätigungselemente (20, 35) zum elektrischen Schalten und/oder zum Ein- bzw. Ausschalten eines Spül- oder Saugvorganges und/oder zur mechanischen Betätigung eines oder mehrerer vom distalen Ende des Rohres (14) vorstehenden Arbeitswerkzeuge (26', 46) befinden, wobei insbesondere zwischen dem Instrumentensockel (12) und dem relativ dazu drehbaren Instrumentenkörper (13), insbesondere dem Anschlußkörper (17) Schleifer (21)-Schleifring (22)-Kontakte und/oder entsprechend der maximal vorkommenden Relativverdrehung biegsame, elektrische und/oder hydraulische und/oder pneumatische Leitungsanordnungen vorgesehen sind, und/oder
daß von elektrischen Betätigungselementen (20) am Handgriff (11) bzw. Instrumentensockel (12) Steuerleitungen über die Schleifer(21) - Schleifring(22) - Kontakte und/oder biegsame Leitungsstücke zum Anschlußkörper (17) verlaufen, und/oder
daß an der Rückseite des Bedienungshandgriffes (11) oben - mit dem Daumen einer den Bedienungshandgriff (11) haltenden Hand erreichbar - elektrische Betätigungselemente (20) in Form einer Tastatur (57) vorgesehen sind.

6. Multifunktionsinstrument nach einem der vorhergehenden Ansprüche,
dadurch **gekennzeichnet,**
daß das Arbeitsinstrument (24) außer der Arbeitsposition, in der das Arbeitswerkzeug (46) über das distale Ende des Rohres (14) vorsteht, eine zurückgezogene Ruheposition aufweist, in der das Arbeitswerkzeug (46) in das Innere des Rohres (14) zurückgezogen ist, wobei insbesondere das Arbeitsinstrument in der vorgeschobenen Arbeitsposition verrastet und durch Federkraft (51) aus der Arbeitsposition in Richtung der zurückgezogenen Ruheposition vorgespannt ist, wobei die Verrastung vorzugsweise mittels eines Auslösers (41) lösbar ist und wobei weiter vorzugsweise die Federkraft durch eine Rückstellfederanordnung (51) erzeugt ist, welche in der Ruheposition entspannt und in der Arbeitsposition gespannt ist.

7. Multifunktionsinstrument nach einem der vorhergehenden Ansprüche,
dadurch **gekennzeichnet,**
daß das Arbeitsinstrument (24) innerhalb der Arbeitskanalanordnung (23, 23', 23'', 23''', 23'''') vorzugsweise um mehr als 360° verdrehbar ist und/oder das Arbeitsinstrument (24) im proximalen Endbereich ein mit dem Instrumentensockel (12) axial verrastbares Basisteil (47) aufweist, welches vorne ein zur Betätigung eines zumindest zwei Arbeitsstellungen aufweisenden Arbeitswerkzeuges (46) vorzugsweise gegen Federkraft (48) axial, insbesondere nach hinten verschiebbares Bauelement (44) trägt, wobei vorzugsweise am Instrumentensockel (12) ein nach außen vorstehender Betätigungshebel (35) vorzugsweise axial verschiebbar angeordnet ist, welcher bei eingeschobenem Arbeitsinstrument (24) mit dem Bauelement (44) derart in vorzugsweise axial festen Eingriff bringbar ist, daß insbesondere durch Zurückziehen des Betätigungshebels (35) das Bauelement (44) relativ zu dem am Instrumentensockel (12) axial festgelegten Basisteil nach hinten verschoben und dadurch das Arbeitswerkzeug (46) betätigt werden kann.

8. Multifunktionsinstrument nach einem der vorhergehenden Ansprüche,
dadurch **gekennzeichnet,**
daß das Arbeitsinstrument (24) als relativ zum Basisteil (47) verschiebbares Bauelement ein vorzugsweise einen mit dem Basisteil (47) fest verbundenen bzw. einstückigen Arbeitsstab (45) mit dem Arbeitswerkzeug (46) an seinem distalen Ende axial relativ verschiebbar enthaltendes Führungsrohr (44) aufweist, welches in der vorgeschobenen Arbeitsposition des Arbeitsinstrumentes (24) relativ zum Rohr (14) und zum Arbeitsstab (45) zwischen zwei axialen Positionen verschiebbar ist, wodurch das Arbeitswerkzeug (46) zwischen einer aktiven und einer inaktiven Stellung, z. B. einer Klemm- und einer Nicht-Klemmstellung verstellt werden kann, wobei insbesondere am vorderen Ende des Arbeitsstabes (45) metallisch leitende Elektroden (46', 46''), die vorzugsweise mit einem Hochfrequenz-Koagulationsstrom gespeist sind, vorgesehen sind, welche durch das vorzugsweise federnd (48) vorgeschobene Führungsrohr (44) gegeneinander geklemmt und durch Zurückziehen des Führungsrohres (44) entklemmt werden.

9. Multifunktionsinstrument nach einem der vorhergehenden Ansprüche,
dadurch **gekennzeichnet,**
daß neben einer Verlängerung (23''') des Arbeitskanals (23) im Instrumentensockel (12) ein gegen eine Federkraft (39) quer verschiebliches Rastelement (38) vorgesehen ist, welches bei eingeschobenem Arbeitsinstrument (24) mit diesem in einem axial festhaltenden Rasteingriff tritt.

10. Multifunktionsinstrument nach einem der Ansprüche 7 bis 9,
dadurch **gekennzeichnet,**
daß der Betätigungshebel (35) eine Bohrung (49) für die axiale Durchführung des Führungsrohres (44) und das Führungsrohr (44) hinter dem Betätigungshebel (35) einen Anschlagkragen (50) für den Betätigungshebel (35) aufweist, wobei vorzugsweise zwischen dem Anschlagkragen (50) und dem Basisteil (47) ein für die Betätigungsbewegung des Führungsrohres (44) ausreichender Abstand (A) vorgesehen ist, und/oder daß im Instrumentensockel (12) ein gegen eine Federkraft (51) axial nach vorne verschiebbarer Anschlagkragen-Eingriffshalter (52) vorgesehen ist, der aufgrund der Federkraft (51) mit der vorderen Fläche des Betätigungshebels (35) in axialem Kraft- oder insbesondere formschlüssigen Eingriff steht, wobei vorzugsweise der Anschlagkragen-Eingriffshalter (52) bei eingeschobenem Arbeitsinstrument (24) gegebenenfalls über einen Fortsatz (52') vom Basisteil (47) bzw. dessen Rastkragen (36) von hinten kraft-, insbesondere formschlüssig beaufschlagt ist, und zwar unmittelbar nach dem der Anschlagkragen (50) von hinten mit dem am Anschlagkragen-Eingriffshalter (52) anliegenden Betätigungshebel (35) in Eingriff gekommen ist.

11. Multifunktionsinstrument nach einem der vorhergehenden Ansprüche,
dadurch **gekennzeichnet,**
daß der Exzenter (16) am Anschlußkörper (17) um die exzentrische Achse (42) des Arbeitskanals (23) und der damit axial ausgerichteten Verlängerungen (23'', 23''') im Exzenter (16) drehgeführt und innerhalb des Instrumentensockels (12) gegen Verdrehung im wesentlichen festgehalten ist, wobei zwischen dem Exzenter (16) und den ihn umgehenden Wänden des Instrumentensockels (12) ausreichenden Spielraum (66) vorhanden ist, damit der Exzenter (16) die bei Drehung des Instrumentenkörpers (13) auftretende Taumelbewegung um die Längsmittelachse (15) des Rohres (14) mitmachen kann.

12. Multifunktionsinstrument nach einem der vorhergehenden Ansprüche,
dadurch **gekennzeichnet,**
daß die Festlegungs- bzw. Verrastungsmittel (38, 39, 40) und die Betätigungsmittel (35, 51, 52) für das Arbeitsinstrument (24) und gegebenenfalls deren Führungsmittel (56) im entsprechend hohl ausgebildeten Exzenter (16) vorgesehen sind.

13. Multifunktionsinstrument nach einem der vorhergehenden Ansprüche,
dadurch **gekennzeichnet,**
daß der Instrumentenkörper (13) vom Instrumentensockel lösbar ausgebildet ist und/oder der Instrumentenkörper (13) zwei Gehäusehalbschalen (17a, 17b) besitzt, die durch ein oder mehrere Verbindungselemente, die den Instrumentensockel (12) und/oder einen Spannring, und/oder eine Schraubkappe (28), vorzugsweise lösbar zusammengehalten und drehbar sowie axial fest, jedoch bevorzugt lösbar mit dem Instrumentensockel (12) verbunden sind, wobei insbesondere die Gehäusehalbschalen (17a, 17b) zum Instrumentensockel (12) hin vorstehende Zapfenteile (67, 68) aufweisen, die in eine dazu komplementäre kreisrunde Öffnung (69) am vorderen Ende des Instrumentensockels (12) eingreifen und dort drehbar, aber axial fest angebracht sind, und/oder in den beiden Gehäusehalbschalen (17a, 17b) ein Anschlußteil (33) angeordnet ist, an bzw. in dem gegebenenfalls die Schleifkontakte (21) und/oder die Fluid-Ver- bzw. -Entsorgungsleitungen (18) und/oder die elektrischen Versorgungs- und Steuerleitungen (19) und/oder einer Axialbohrung (34) für das Rohr (14) sowie die Verlängerung (23') des Arbeitskanals (23) und/oder die zur Drehachse (15) exzentrische Aufnahmebohrung (70) für einen zur Achse (42) des Arbeitskanals (23) zentrischen Führungszapfen (71) des Exzenters (16) vorgesehen sind, wobei gegebenenfalls in einer (17b) der Gehäusehalbschalen (17a, 17b) Durchlaßschlitze (72, 73) für die Fluid-Versorgungs- bzw. -Entsorgungsleitungen (18) und/oder die elektrischen Versorgungs- und/oder -Steuerleitungen (19) vorgesehen sind.

14. Multifunktionsinstrument nach einem der vorhergehenden Ansprüche,
dadurch **gekennzeichnet,**
daß im Rohr (14) und im Anschlußkörper (17) ein separater kleinlumiger Flüssigkeits-Spül- oder Saugkanal (74) vorgesehen ist, der im Anschlußkörper (17) an die Fluid-Ver- bzw. -Entsorgungsleitungen (18) angeschlossen ist.

15. Multifunktionsinstrument nach einem der vorhergehenden Ansprüche,
dadurch **gekennzeichnet,**
daß der Arbeitskanal (23) bei herausgenommenen Arbeitsinstrument (24) als Flüssigkeits-Spül- oder Saugkanal (74) verwendet wird, indem er hinten dicht verschließbar (82) und an die Fluid-Ver- bzw. Entsorgungsleitungen (18) anschließbar ist, wobei insbesondere im Anschlußkörper (17) ein Umschaltventil (75) vorgesehen ist, welches von einem am Arbeitskanal (23) angeordneten Betätigungselement (76) derart gesteuert wird, daß es bei in die Verlängerung (23') des Arbeitskanals (23) im Anschlußkörper (17) eingeschobenem Arbeitsinstrument (14) den kleinlumigen Flüssigkeits-Spül- oder Saugkanal (74) gegebenenfalls über eine Anschlußleitung (78) an die Fluid-Ver- bzw. -Entsorgungsleitungen (18) und bei aus der Verlängerung (23') herausgezogenem Arbeitsinstrument (24) den Arbeitskanal (23) gegebenenfalls über eine Anschlußleitung (77) an die Fluid-Ver- bzw. -Entsorgungsleitungen (18) anschließt.

16. Multifunktionsinstrument nach einem der vorhergehenden Ansprüche,
dadurch **gekennzeichnet,**
daß neben dem exzentrisch angeordneten großlumigen Arbeitskanal (23) ein vorzugsweise exzentrisch angeordneter, insbesondere einen kleineren Querschnitt aufweisender zweiter Arbeitskanal (29) vorgesehen ist, in dem ein weiteres stabförmiges Instrument, insbesondere ein Hochfrequenz-Schneid- oder Koagulationsinstrument (26) mit seiner Arbeitselektrode (29) aus dem distalen Ende des Rohres (14) ausfahrbar bzw. in dieses zurückziehbar ausgebildet ist.

17. Multifunktionsinstrument nach Anspruch 16,
dadurch **gekennzeichnet,**
daß der zweite Arbeitskanal (29) bis in den Anschlußkörper (17) reicht und daß dort ein von Hand steuerbares mechanisches Betätigungselement oder bevorzugt ein von einem äußeren Betätigungselement (20) steuerbarer Antrieb (79) vorgesehen ist, der das Arbeitswerkzeug, insbesondere die Arbeitselektrode (26') in Abhängigkeit vom Drücken zugeordneter Betätigungselemente (20) der Tastatur (57) ein- und ausfährt.

## Claims

1. Multifunctional instrument for performing endoscopic operations having an instrument socket (12) carrying an operator hand grip (11), wherein an instrument body (13) is mounted at the instrument socket (12) and comprises a multilumen tube (14) which is guidable through a trocar into a body cavity of a patient, preferably consists of electrically non-conductive material, and has at least one axial working passage (23), which is axially extended by axial extensions (23', 23'', 23''') in the instrument socket (12), forming complementary passages into which a rod-like working instrument (24) having a working tool (46) provided at the distal end and a proximal connection or actuating end (43) at the rear, which preferably projects from the instrument socket (12), can be inserted, preferably from the rear, and fixed in the working position, with the longitudinal central axis (42) of the working tool being eccentrically arranged relative to the central axis (15) of the tube (14), characterized in that the instrument body (13) is rotatable about one of its longitudinal axes, preferably the central axis (15) of the tube (14), relative to the instrument socket (12) which is firmly connected to the operator hand grip (11) and can be held in the set rotational position; and in that an axial extension (23'', 23''') of the working passage (23) is eccentrically arranged in an eccentric member (16), which is guidable within the instrument socket (12) with radial clearance (66), together with the axis (42) of the extensions (23'', 23''') about the axis of rotation (15) of the instrument body (13) around the axis (42) of the working passage (23), but axially fixedly arranged, such that the extensions (23'', 23''') in the eccentric member (16) can be brought in each rotational position of the instrument body (13) relative to the instrument socket (12) into axial alignment with the working passage (23) or its extension (23') in the connection body (17), with the extensions (23'', 23''') provided in the eccentric member (16) being accessible in each rotary position of the eccentric member (16) through an opening (23'''') in the rear end face of the instrument socket (12), and wherein the axis (42) of the working passage (23), on the one hand, and the axis of the extensions (23'', 23''') in the eccentric member (16), on the other hand, are held in axial alignment in each rotary position of the instrument body (13).

2. Multifunctional instrument in accordance with claim 1, characterized in that the operator hand grip (11) extends at least substantially transverse to the longitudinal axis (15) of the instrument body (13), i.e. of the tube (14).

3. Multifunctional instrument in accordance with claim 1 or claim 2, characterized in that the rotational connection between the instrument socket (12) and the instrument body (13) is a stiff one, such that a rotational movement is possible, but such that the selected rotational position is retained when used for the intended purpose.

4. Multifunctional instrument in accordance with one of the preceding claims, characterized in that the instrument body (13) has a connection body (17) rotatably mounted at the instrument socket (12), and from which supply and removal lines and/or control lines (18, 19) branch off, preferably laterally, and has the preferably releasably mounted tube (14) at its end remote from the instrument socket (12); and in that an axial extension (23') of the working passage (23) in the connection body (17) of the instrument body (13) is eccentrically arranged relative to the axial axis of rotation (15) of the connection body (17), i.e. of the tube (14), with the connection body (17) and the eccentric member (16) preferably being so rotationally coupled that the extensions (23', 23'', 23''') of the working passage (23) provided in them are always axially aligned.

5. Multifunctional instrument in accordance with one of the preceding claims, characterized in that actuation elements (20, 35) for electrical switching and/or for the switching on or off of a flushing or suction process and/or for the mechanical actuation of one or more working tools (26', 46) projecting from the distal end of the tube (14) are located at the operator hand grip (11), or at the instrument socket (12), and can be operated without loosening of the firm grip, for example with the thumb or index finger, wherein a slipper (21) - slip ring (22) contacts and/or electrical and/or hydraulic and/or pneumatic line arrangements, flexible in accordance with the maximum relative rotation which occurs, are provided in particular between the instrument socket (12) and the instrument body (13) rotatable relative thereto, in particular between the instrument socket (12) and the connection body (17), and/or in that control lines pass via the slipper (21) - slip ring (22) contacts and/or flexible line elements to the connection body (17) from electrical actuating elements (20) at the hand grip (11) or at the instrument socket (12); and/or in that electrical actuating elements (20) in the form of a keyboard (57) are provided at the rear side of the operator hand grip (11) at the top - reachable with the thumb of a hand holding the operator hand grip (11).

6. Multifunctional instrument in accordance with one of the preceding claims, characterized in that the working instrument (24) has a retracted rest position in which the working tool (46) is retracted into the interior of the tube (14) in addition to the working position in which the working tool (46) projects beyond the distal end of the tube (14), with the working instrument in particular latching in the advanced working position and being biased by a spring force (51) out of the working position in the direction towards the retracted rest position, wherein the latching is preferably releasable by means of a trigger (41), and wherein furthermore the spring force is preferably generated by a resetting spring arrangement (51), which is relaxed in the rest position and stressed in the working position.

7. Multifunctional instrument in accordance with one of the preceding claims, characterized in that the working instrument (24) is rotatable within the working passage arrangement (23, 23', 23'', 23''', 23''''), preferably through more than 360°, and/or that the working instrument (24) has a base part (47) in the proximal end region which can be axially latched to the instrument socket (12), and which carries at the front an axially and in particular rearwardly shiftable component (44), which is preferably shiftable against spring force, for the actuation of a working tool (46) having at least two working positions, wherein an outwardly projecting actuating lever (35) is preferably axially shiftably arranged at the instrument socket (12) and can be brought into firm engagement with the component (44), preferably axially firm engagement, when the working instrument (24) is inserted so that the component (44) can be shifted rearwardly relative to the base part axially fixed at the instrument socket (12), in particular by retraction of the actuating lever (35), and can thereby actuate the working tool (46).

8. Multifunctional instrument in accordance with one of the preceding claims, characterized in that the working instrument (24) has a guide tube (44) as the component which is shiftable relative to the base part (47), the guide table (44) containing an axially relatively shiftable working rod (45) with the working tool (46) at its distal end and the working rod (45) being preferably firmly connected to the base part (47) or in one piece therewith, with the guide tube being shiftable in the advanced working position of the working instrument (24) relative to the tube (14) and to the working rod (45) between two axial positions, whereby the working tool (46) can be displaced between an active position and an inactive position, for example between a clamping position and a non-clamping position, wherein metallically conductive electrodes (46', 46'') are provided, in particular at the front end of the working rod (45), which are fed preferably with a high frequency coagulation current, which are clamped against one another by the preferably resiliently (48) advanced guide tube (44) and can be unclamped by retraction of the guide tube (44).

9. Multifunctional instrument in accordance with one of the preceding claims, characterized in that a latch element (38), which is transversely displaceable against a spring force (39), is provided in the instrument socket (12) alongside an extension (23''') of the working passage (23) and enters, with the working instrument (24) inserted, into an axially firmly holding latched engagement with the latter.

10. Multifunctional instrument in accordance with one of the claims 7 to 9, characterized in that the actuating lever (35) has a bore (49) for the axial passage of the guide tube (44), and the guide tube (44) has an abutment collar (50) behind the actuating lever (35) for the actuating lever (35), wherein a spacing (A), which is sufficient for the actuating movement of the guide tube (44), is preferably provided between the abutment collar (50) and the base part (47); and/or in that an engagement holder (52) for the abutment collar which can be shifted axially forwardly against a spring force, is provided in the instrument socket (12) and stands, as a result of the spring force (51), in axially force transmitting and in particular form locked engagement, with the front surface of the actuating lever (35), with the abutment collar engagement holder (52) preferably being acted on from the rear in force transmitting and in particular in form locked manner when the working instrument (24) has been inserted, optionally via a projection (52'), from the base part (47) or its latching collar (36); and indeed directly after the abutment collar (50) has come into engagement from the rear with the actuating lever (35) contacting the abutment collar engagement holder (52).

11. Multifunctional instrument in accordance with one of the preceding claims, characterized in that the eccentric member (16) at the connection body (17) is rotatably guided around the eccentric axis (42) of the working passage (23) and of the extensions (23'', 23''') axially aligned therewith in the eccentric member (16), and is substantially firmly held against rotation within the instrument socket (12), wherein an adequate clearance (66) is present between the eccentric member (16) and the walls of the instrument socket (12) surrounding it, so that the eccentric member (16) can participate in the transport movement around the longitudinal axis (15) of the tube (14) which occurs on rotation of the instrument body (13).

12. Multifunctional instrument in accordance with one of the preceding claims, characterized in that the fixing means and latching means (38, 39, 40) and the actuating means (35, 51, 52) for the working instrument (24), and optionally their guide means (56), are provided in the correspondingly hollow shaped eccentric member (16).

13. Multifunctional instrument in accordance with one of the preceding claims, characterized in that the instrument body (13) is made releasable from the instrument socket and/or the instrument body (13) has two housing half shells (17a, 17b) which are preferably releasably held together by one or more connection elements comprising the instrument socket (12) and/or a clamping ring and/or a screw cap (28) and which are rotatably but axially fixedly and preferably releasably connected to the instrument socket (12), wherein the housing half shells (17a, 17b) in particular have spigot parts (67, 68) projecting towards the instrument socket (12), which engage in a complementary circular opening (69) provided at the front end of the instrument socket (12) and are rotatably but axially fixedly mounted there; and/or in that a connection part (33) is arranged in the two housing half shells (17a, 17b), at which or in which the slip contacts (21) and/or the liquid supply and discharge lines (18) and/or the electrical supply and control lines (19) and/or an axial bore (34) for the tube (14) and also the extension (23') of the working passage (23) and/or the receiving bore (70) eccentric to the axis of rotation (15) for a guide spigot (71) of the eccentric member (16) concentric to the axis (42) of the working passage (23) are optionally provided, wherein passage slots (72, 73) for the liquid supply and discharge lines (18) and/or the electrical supply lines and/or control lines (19) are optionally provided in one (17b) of the housing half shells (17a, 17b).

14. Multifunctional instrument in accordance with one of the preceding claims, characterized in that a separate, small lumen liquid flushing or suction passage (74) is provided in the tube (14) and in the connection body (17), which is connected in the connection body (17) to the liquid supply and/or discharge lines (18).

15. Multifunctional instrument in accordance with one of the preceding claims, characterized in that the working passage (23) is used when the working instrument (24) is extracted as a liquid flushing or suction passage (74), in that it is sealingly closable (82) at the rear and connectable to the liquid supply and/or discharge lines (18), wherein a changeover valve (75) is provided, in particular in the connection body (17), and is controlled from an actuating element (76) arranged at the working passage (23) in such a way that, when the working instrument (14) is inserted into the extension (23') of the working passage (23) in the connection body (17), it connects the small lumen liquid flushing or suction passage (74) to the fluid supply and/or discharge lines (18) optionally via a connection line (78) and, when the working instrument (24) is extracted from the extension (23'), connects the working passage (23) to the fluid supply and/or discharge lines (18), via a connection line (77), if required.

16. Multifunctional instrument in accordance with one of the preceding claims, characterized in that a preferably eccentrically disposed second working passage (29), in particular having a smaller cross-section, is provided adjacent to the eccentrically arranged, large lumen working passage (23), with a working electrode (29) of a further, rod-like instrument within the second working passage (29), in particular a high frequency cutting or coagulation instrument (26), being made extendible out of the distal end of the tube (14) or retractable into the latter.

17. Multifunctional instrument in accordance with claim 16, characterized in that the second working passage (29) extends up to an and into the connection body (17), and in that a mechanical actuating element controllable by hand or preferably a drive (79) controllable by an outer actuating element (20) is provided there, which moves the working tool, in particular the working electrode (26') inwardly and outwardly in dependence on the pressing of associated actuating elements (20) of the keyboard (57).

## Revendications

1. Instrument à fonctions multiples utilisé pour des opérations endoscopiques comportant un socle (12) d'instrument portant une poignée de service (11), sur lequel est monté un corps d'instrument (13) comportant un tube (14) à plusieurs lumières susceptible d'être introduit dans une cavité corporelle d'un patient au moyen d'un trocart, qui est constitué de préférence d'un matériau non conducteur de l'électricité et qui présente au moins un canal de travail axial (23) qui se prolonge axialement par des prolongements axiaux formant des canaux complémentaires (23', 23'', 23''') dans le socle (12) de l'instrument, dans lequel canal de travail peut être enfilé, de préférence à partir de l'arrière, un instrument de travail (24) en forme de barre comportant un outil de travail (46) prévu à l'extrémité distale et une extrémité (43) proximale de raccordement, ou d'actionnement, faisant de préférence saillie à l'arrière, à partir du socle (12) de l'instrument, et lequel instrument de travail peut être fixé en position de travail, l'axe longitudinal médian (42) du canal de travail étant excentrique par rapport à l'axe médian (15) du tube (14),
caractérisé en ce que
le corps (13) de l'instrument peut tourner sur un de ses axes longitudinaux, de préférence l'axe médian (15) du tube (14) par rapport au socle (12) de l'instrument relié de façon fixe à la poignée de service (11) et peut être maintenu dans la position de rotation réglée, et en ce qu'un prolongement axial (23'', 23''') du canal de travail (23) est disposé dans un excentrique (16) mis en place à l'intérieur du socle (12) de l'instrument avec un jeu radial (66), mais en étant cependant fixe axialement, susceptible d'être tourné avec l'axe (42) des prolongements (23'', 23''') autour de l'axe de rotation (15) du corps (13) de l'instrument, en étant cependant sensiblement fixe en rotation par rapport à l'axe (42) du canal de travail (23) d'une façon excentriquement telle que les prolongements (23'', 23''') présents dans l'excentrique (16) peuvent être amenés, pour chaque position de rotation du corps (13) de l'instrument par rapport au socle (12) de l'instrument, en alignement axial avec le canal de travail (23), ou avec son prolongement (23') dans le corps (17) de raccordement, les prolongements (23'', 23''') prévus dans l'excentrique (16) étant accessibles dans chaque position de rotation de l'excentrique (16) par une ouverture (23'''') présente dans la face arrière du socle (12) de l'instrument et l'axe (42) du canal de travail (23) d'une part, ainsi que l'axe des prolongements (23'', 23''') présents dans l'excentrique (16) d'autre part, étant maintenus en alignement axial pour chaque position de rotation du corps (13) de l'instrument.

2. Instrument à fonctions multiples selon la revendication 1,
caractérisé en ce que
la poignée de service (11) s'étend au moins sensiblement transversalement à l'axe longitudinal médian (15) du corps de l'instrument (13), ou du tube (14).

3. Instrument à fonctions multiples selon la revendication 1 ou 2,
caractérisé en ce que
la liaison rotative entre le socle (12) de l'instrument et le corps (13) de l'instrument est dure, de façon telle qu'un réglage rotatif est possible, mais que cependant une position de rotation une fois réglée est maintenue dans le cas d'une utilisation telle que prévu.

4. Instrument à fonctions multiples selon l'une des revendications précédentes,
caractérisé en ce que
le corps (13) de l'instrument présente un corps de raccordement (17), qui est monté de façon rotative sur le socle (12) de l'instrument et duquel partent, de préférence latéralement, des conduits d'alimentation ou d'évacuation et/ou des conduits de commande (18, 19), et présente, à son extrémité située à l'opposé du socle (12) de l'instrument, le tube (14) monté de préférence de façon à pouvoir être démonté, et en ce qu'un prolongement axial (23') du canal de travail (23) présent dans le corps (17) de raccordement du corps (13) de l'instrument est disposé de façon excentrique par rapport à l'axe de rotation axial (15) du corps de raccordement (17), ou du tube (14), le corps de raccordement (17) et l'excentrique (16) étant de préférence couplés en rotation, de façon telle que les prolongements (23', 23'', 23''') du canal de travail (23) prévus dans ceux-ci sont en permanence alignés axialement.

5. Instrument à fonctions multiples selon l'une des revendications précédentes,
caractérisé en ce que
sur la poignée de service (11) ou sur le socle (12) de l'instrument, se trouvent des éléments d'actionnement (20, 35) utilisables sans relâcher la poignée fixe, par exemple avec le pouce, ou avec l'index, en vue du branchement électrique et/ou en vue de la mise en marche ou de l'arrêt d'un processus de rinçage ou d'aspiration et/ou en vue de l'actionnement mécanique d'un ou de plusieurs outils de travail (26', 46) qui font saillie à partir de l'extrémité distale du tube (14), des contacts à frotteur (21) et à bague de contact (22) et/ou des dispositifs de conduits électriques et/ou hydrauliques et/ou pneumatiques souples en fonction de la rotation relative maximum qui se produit étant prévus en particulier entre le socle (12) de l'instrument et le corps (13) de l'instrument susceptible de tourner par rapport à celui-ci, en particulier le corps de raccordement (17),
et/ou en ce que des conduits de commande s'étendent à partir d'éléments d'actionnement électriques (20) présents sur la poignée (11) ou sur le socle (12) de l'instrument en passant par des contacts à frotteur (21) et bague de contact (22) et/ou des tronçons de conduits souples jusqu'au corps de raccordement (17),
et/ou en ce que sur le côté arrière de la poignée de service (11), en haut, des éléments d'actionnement électriques (20) sont prévus sous la forme d'un clavier (57), lesquels éléments d'actionnement sont susceptibles d'être atteints avec le pouce d'une main qui tient la poignée de service (11).

6. Instrument à fonctions multiples selon l'une des revendications précédentes,
caractérisé en ce que
l'instrument de travail (24) présente, en plus de la position de travail dans laquelle l'outil de travail (46) fait saillie au-delà de l'extrémité distale du tube (14), une position de repos en retrait dans laquelle l'outil de travail (46) est en retrait dans l'intérieur du tube (14), en particulier l'instrument de travail s'encliquetant dans la position de travail en saillie et étant sous précontrainte, par l'effet de la force élastique (51) hors de la position de travail en direction de la position de repos en retrait, l'encliquetage pouvant être libéré de préférence au moyen d'un déclencheur (41) et, en outre, de préférence la force élastique étant produite par un dispositif (51) à ressort de rappel qui est détendu dans la position de repos et qui est tendu dans la position de travail.

7. Instrument à fonctions multiples selon l'une des revendications précédentes,
caractérisé en ce que
l'instrument de travail (24) peut être tourné de préférence de plus de 360° à l'intérieur du dispositif de canal de travail (23, 23', 23'', 23''', 23'''') et/ou l'instrument de travail (24) présente, dans la zone d'extrémité proximale, une pièce de base (47) qui est susceptible d'être encliquetée axialement avec le socle (12) de l'instrument et qui porte à l'avant, en vue de l'actionnement d'un outil de travail (46) présentant au moins deux positions de travail, un élément constitutif (44) susceptible d'être déplacé par translation, de préférence axialement, à l'encontre d'une force élastique (48), en particulier vers l'arrière, un levier d'actionnement (35) faisant saillie vers l'extérieur étant disposé, de préférence sur le socle (12) de l'instrument, de façon à pouvoir être déplacé axialement, lequel levier d'actionnement pouvant être amené en prise fixe, de préférence axialement, avec l'élément constitutif (44), l'instrument de travail (24) étant rentré, de façon telle qu'en particulier en tirant en arrière le levier d'actionnement (35) l'élément constitutif (44) peut être déplacé vers l'arrière par rapport à la pièce de base fixée axialement par rapport au socle (12) de l'instrument, et, de ce fait, l'outil de travail (46) pouvant être actionné.

8. Instrument à fonctions multiples selon l'une des revendicatons précédentes,
caractérisé en ce que
l'instrument de travail (24) présente, en tant qu'élément constitutif susceptible d'être déplacé par rapport à la pièce de base (47), un tube de guidage (44) renfermant de préférence une tige de travail (45) fixée à la pièce de base (47) ou formant une seule pièce avec celle-ci, en étant déplaçable axialement de façon relative avec l'outil de travail (46) à son extrémité distale, lequel tube de guidage pouvant, l'instrument de travail (24) étant en position de travail en saillie, être déplacé par rapport au tube (14) et à la tige de travail (45) entre deux positions axiales, grâce à quoi l'outil de travail (46) peut être déplacé entre une position active et une position inactive, par exemple une position de pincement et une position de non-pincement, des électrodes métalliques conductrices (46', 46'') étant en particulier prévues à l'extrémité avant de la tige de travail (45), qui sont de préférence alimentées par un courant de coagulation à haute fréquence, et qui sont serrées l'une contre l'autre par le tube de guidage (44) poussé vers l'avant, de préférence élastiquement (48), et desserrées grâce au retrait du tube de guidage (44).

9. Instrument à fonctions multiples selon l'une des revendications précédentes,
caractérisé en ce que
en plus d'un prolongement (23''') du canal de travail (23), est prévu dans le socle (12) de l'instrument, un élément d'encliquetage (38) qui est susceptible d'être déplacé transversalement à l'encontre d'une force élastique (39) et qui, lorsque l'instrument de travail (24) est rentré, se met en prise d'encliquetage fixe axialement avec celui-ci.

10. Instrument à fonctions multiples selon l'une des revendications 7 à 9,
caractérisé en ce que
le levier d'actionnement (35) présente un alésage (49) destiné au passage axial du tube de guidage (44) et le tube de guidage (44) présente derrière le levier d'actionnement (35) un collet de butée (50) pour le levier d'actionnement (35), une distance suffisante (A) étant prévue de préférence entre le collet de butée (50) et la pièce de base (47) pour le mouvement d'actionnement du tube de guidage (44), et/ou en ce que dans le socle (12) de l'instrument est prévu un élément (52) de maintien en prise du collet de butée susceptible d'être déplacé axialement vers l'avant à l'encontre d'une force élastique (51) et qui, en raison de la force élastique (51), est axialement en prise par force, ou en particulier en prise par complémentarité de formes avec la surface avant du levier d'actionnement (35), l'élément (52) de maintien en prise du collet de butée, l'instrument de travail (24) étant rentré, étant de préférence, le cas échéant, placé, par l'intermédiaire d'un prolongement (52'), sous la contrainte de la pièce de base (47), ou de son collet d'encliquetage (36), par l'arrière, la contrainte étant par force, en particulier par complémentarité de formes, et ceci immédiatement après que le collet de butée (50) est venu en prise à partir de l'arrière avec le levier d'actionnement (35) portant sur l'élément (52) de maintien en prise du collet de butée.

11. Instrument à fonctions multiples selon l'une des revendications précédentes,
caractérisé en ce que
l'excentrique (16) est guidé de façon rotative dans le corps de raccordement (17) autour de l'axe excentrique (42) du canal de travail (23) et des prolongements (23'', 23''') présents dans l'excentrique (16), orientés axialement de ce fait, et est sensiblement maintenu fixe à l'encontre de la rotation à l'intérieur du socle (12) de l'instrument, un jeu (66) suffisant existant entre l'excentrique (16) et les parois du socle (12) de l'instrument qui l'entourent, afin que l'excentrique (16) puisse, lors de la rotation du corps (13) de l'instrument, accompagner le mouvement de nutation qui se produit, autour de l'axe longitudinal médian (15) du tube (14).

12. Instrument à fonctions multiples selon l'une des revendications précédentes,
caractérisé en ce que
les moyens de fixation ou d'encliquetage (38, 39, 40) et les moyens d'actionnement (35, 51, 52) destinés à l'instrument de travail (24) et, le cas échéant, leurs moyens de guidage (56) sont prévus dans l'excentrique (16) conformé en creux de façon correspondante.

13. Instrument à fonctions multiples selon l'une des revendications précédentes,
caractérisé en ce que
le corps (13) de l'instrument est conformé de façon à pouvoir être détaché du socle de l'instrument et/ou le corps (13) de l'instrument comporte deux demi-coques (17a, 17b) de corps qui sont maintenues ensemble, de préférence de façon détachable, par un ou plusieurs éléments de liaison, qui maintiennent assemblés le socle (12) de l'instrument et/ou un collier de serrage et/ou un bouchon fileté (28), et qui sont reliées au socle (12) de l'instrument de façon fixe aussi bien en rotation qu'axialement, mais cependant de préférence de façon détachable, en particulier les demi-coques (17a, 17b) de corps présentant des parties (67, 68) formant des tourillons faisant saillie en direction du socle (12) de l'instrument, qui pénètrent dans une ouverture (69) circulaire de forme complémentaire située à l'extrémité avant du socle (12) de l'instrument et y sont montées de façon rotative mais fixe dans le sens axial, et/ou une pièce de raccordement (33) étant disposée dans les deux demi-coques (17a, 17b) de corps, sur laquelle ou dans laquelle sont prévus, le cas échéant, les contacts frotteurs (21) et/ou les conduits (18) d'alimentation en fluides ou d'évacuation de fluides et/ou les conducteurs (19) électriques d'alimentation et de commande et/ou un alésage axial (34) destiné au tube (14) ainsi qu'au prolongement (23') du canal de travail (23) et/ou l'alésage (70) de logement, excentrique par rapport à l'axe de rotation (15), destiné à un tourillon de guidage (71) de l'excentrique (16), ce tourillon étant centré par rapport à l'axe (42) du canal de travail (23), des fentes de passage (72, 73) étant, le cas échéant, prévues dans une (17b) des demi-coques (17a, 17b) de corps pour les conduits (18) d'alimentation en fluides ou d'évacuation de fluides et/ou les conducteurs (19) électriques d'alimentation et/ou de commande.

14. Instrument à fonctions multiples selon l'une des revendications précédentes,
caractérisé en ce que
est prévu dans le tube (14) et dans le corps de raccordement (17) un canal séparé (74) de rinçage ou d'aspiration de liquides, présentant une lumière de petite dimension, qui est raccordé dans le corps de raccordement (17) aux conduits (18) d'alimentation en fluides ou d'évacuation de fluides.

15. Instrument à fonctions multiples selon l'une des revendications précédentes,
caractérisé en ce que
le canal de travail (23) est utilisé en tant que canal (74) de rinçage ou d'aspiration de liquides lorsque l'instrument de travail (24) est retiré, en pouvant être fermé hermétiquement (82) à l'arrière et raccordé aux conduits (18) d'alimentation en fluides ou d'évacuation de fluides, une valve de commutation (75) étant en particulier prévue dans le corps de raccordement (17), laquelle est commandée par un élément d'actionnement (76) disposé sur le canal de travail (23) de telle manière que, lorsque l'instrument de travail (14) est enfilé dans le prolongement (23') du canal de travail (23), dans le corps de raccordement (17), elle raccorde le canal (74) de rinçage ou d'aspiration de liquides présentant une lumière de petite dimension, le cas échéant par l'intermédiaire d'un conduit de raccordement (78), aux conduits (18) d'alimentation en fluides ou d'évacuation de fluides, et lorsque l'instrument de travail (24) est retiré du prolongement (23'), elle raccorde le canal de travail (23), le cas échéant par l'intermédiaire d'un conduit de raccordement (77), aux conduits (18) d'alimentation en fluides ou d'évacuation de fluides.

16. Instrument à fonctions multiples selon l'une des revendications précédentes,
caractérisé en ce que
en plus du canal de travail (23) présentant une lumière de grande dimension, disposé excentriquement, est prévu un deuxième canal de travail (29) disposé de préférence excentriquement, présentant en particulier une section transversale de plus faible dimension, dans lequel un autre instrument en forme de barre, en particulier un instrument (26) de coupe ou de coagulation à haute fréquence est conformé de façon telle que son électrode de travail (29) peut être sortie hors de l'extrémité distale du tube (14), ou rentrée dans celui-ci.

17. Instrument à fonctions multiples selon la revendication 16,
caractérisé en ce que
le deuxième canal de travail (29) s'étend jusque dans le corps de raccordement (17) et qu'il y est prévu un élément d'actionnement mécanique susceptible d'être commandé à la main, ou que, de préférence, il est prévu un dispositif d'entraînement (79) susceptible d'être commandé par un élément d'actionnement extérieur (20), et qui fait rentrer ou sortir l'outil de travail, en particulier l'électrode de travail (26') en fonction de la pression exercée sur des éléments d'actionnement correspondants (20) du clavier (57) .
